# EUROPEAN PATENT APPLICATION

(11) **EP 1 614 750 A1**
(43) Date of publication of application: **11.01.2006**
(21) Application number: 04727713.2
(22) Date of filing: 15.04.2004
(51) Int. Cl.: C12N 15/10, C07K 2/00

(54) **NOVEL FUNCTIONAL PEPTIDE NUCLEIC ACID AND PROCESS FOR PRODUCING THE SAME**

(30) Priority: 15.04.2003 JP 2003144152
(71) Applicant: Credia Japan Co., Ltd., Soraku-gun, Kyoto 619-0273 (JP)
(72) Inventor: IKEDA, Hisafumi, Nagareyama-shi, Chiba 270-0115 (JP); TONOSAKI, Madoka, Kasukabe-shi, Saitama 344-0054 (JP)
(74) Representative: von Füner, Nicolai
(86) International application number: PCT/JP2004/005392
(87) International publication number: WO 2004/092377

(57) **Abstract**

A functional molecule can be introduced extremely rapidly and with superior cost performance by using a method for producing a functional PNA oligomer that comprises synthesizing a PNA oligomer by reacting a PNA monomer unit having a protected adenine, guanine, cytosine or thymine with Boc-lysine(Fmoc)-OH or Fmoc-lysine(Alloc)-OH, followed by introducing a functional molecule having a free carboxylic acid into said PNA oligomer and de-protecting the protecting group. Moreover, Boc-lysine (Fmoc)-OH or Fmoc-lysine(Alloc)-OH can be produced that functions as a compound and precursor PNA monomer unit synthesized according to this method.

## Description

### TECHNICAL FIELD

The present invention relates to a novel production method of functional peptide nucleic acid oligomers and their intermediates that uses lysine.

### BACKGROUND ART

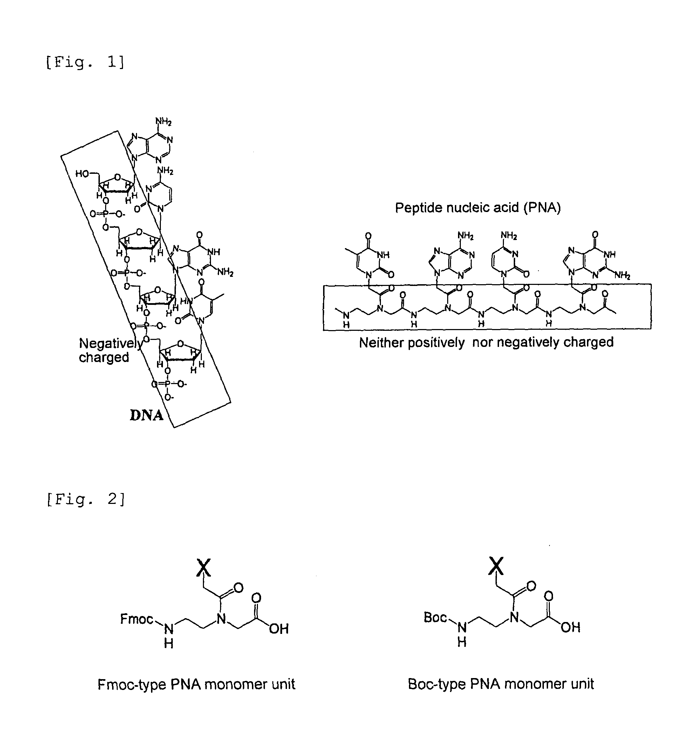

Nucleic acids consist of DNA and RNA that govern the genetic information of living organisms. In contrast, peptide nucleic acids (PNA) refers to modified nucleic acids wherein the sugar-phosphate skeleton of a nucleic acid has been converted to an N-(2-aminoethyl)glycine skeleton (Fig. 1). Although the sugar-phosphate skeletons of DNA/RNA are subjected to a negative charge under neutral conditions resulting in electrostatic repulsion between complementary chains, the backbone structure of PNA does not inherently have a charge. Therefore, there is no electrostatic repulsion. Consequently, PNA has a higher ability to form double strands as compared with conventional nucleic acids, and has a high ability to recognize base sequences. Moreover, since PNA is extremely stable with respect to nucleases and proteases in the living body and is not decomposed by them, studies are being conducted on its application to gene therapy as an antisense molecule.

As a result of using PNA in technology that conventionally used DNA as a medium, it has become possible to compensate for those shortcomings of DNA that were heretofore unable to be overcome. For example, PNA can be applied to "DNA microarray technology" for rapid and large-volume systematic analysis of genetic information, as well as recently developed "molecular beacons" used as probes capable of detecting that a base sequence has been specifically recognized using emission of fluorescent light. Since both of these use DNA lacking enzyme resistance as the medium, strict sampling is required when using these technologies. The satisfying of this requirement is the key to achieving greater sophistication of these technologies.

On the other hand, since PNA is completely resistant to enzymes, by substituting the use of DNA for PNA in DNA microarray technology and molecular beacons, the previously mentioned technical shortcomings can be overcome, leading to expectations of being able to take further advantage of the merits of these technologies.

Although there are many other fields wherein the use of PNA is expected to lead to further advancements in addition to DNA microarray technology and molecular beacons, in these fields it will be necessary to design novel PNA monomers by enabling PNA to function efficiently, namely by realizing the efficient introduction of functional molecules into PNA monomers.

Since ordinary solid-phase peptide synthesis methods are used for PNA oligomer synthesis methods, classification of PNA monomer units according to PNA backbone structure yields the two types consisting of Fmoc type PNA monomer units and Boc type PNA monomer units (Fig. 2).

Methods for synthesizing Fmoc type PNA monomer units have already been established, and since their oligomer synthesis can be carried out using an ordinary DNA automated synthesizer, synthesis can be carried out on a small scale by the following route: 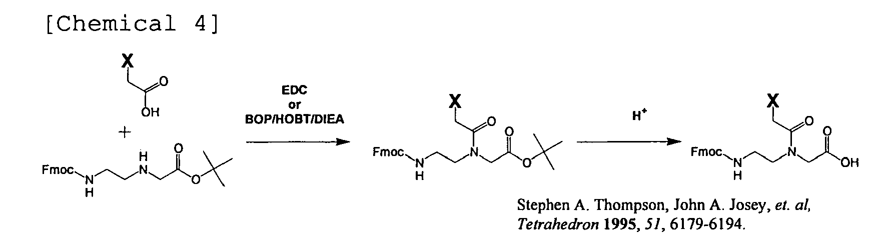 (wherein X represents guanine, thymine, cytosine or adenine).

Furthermore, Fmoc refers to 9-fluorenylmethoxycarbonyl, Boc refers to t-butoxycarbonyl, and Alloc refers to allyloxycarbonyl.

Initially, Boc type PNA monomer units like those shown below: 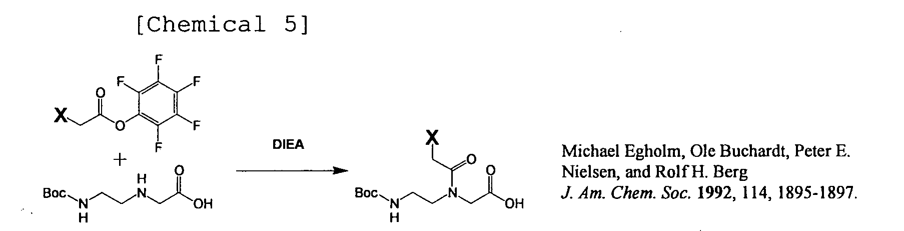 were used and this was followed by the establishment of more efficient synthesis methods. 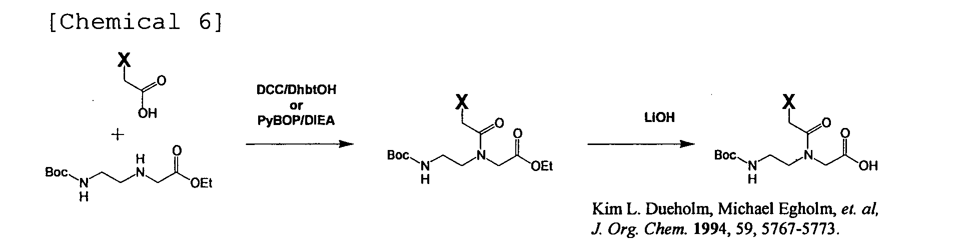 However, since the previously mentioned Fmoc type was developed that offered easier handling, the frequency of use of the Boc type is decreasing.

However, when introducing a functional molecule other than the four types of nucleic acid bases of guanine, thymine, cytosine and adenine, such as when introducing a photofunctional molecule, there are many cases wherein the functional molecule to be introduced is unstable under alkaline conditions, and thus a Boc type of PNA backbone structure that is not used under alkaline conditions is highly useful. A patent application for a "method for producing t-butoxycarbonyl-aminoethylamine and amino acid derivatives" has already been made by the inventors of the present invention as Japanese Patent Application No. 2000-268638.

In addition, there are also five examples of synthesis of monomer units of photofunctional oligo PNA in the prior art. Although all of these use the above route, their yields are either not described or are extremely low (Peter E. Nielsen, Gerald Haaiman, Anne B. Eldrup PCT Int. Appl. (1998) WO 985295 A1 19981126, T.A. Tran, R.H. Mattern, B. A. Morgan (1999) J. Pept. Res, 53, 134-145, Jesper Lohse et al. (1997) Bioconjugate Chem., 8, 503-509, Hans-georg Batz, Henrik Frydenlund Hansen, et al. Pct Int. Appl. (1998) WO 9837232 A2 19980827, Bruce Armitage, Troels Koch, et al. (1998) Nucleic Acid Res., 26, 715-720). In addition, since the structures of the compounds used have the characteristic of being comparatively stable under alkaline conditions, they are expected to be uable to be produced in good yield using a method similar to the above-mentioned methods of the prior art, namely the following route A, if an unstable chromophore attaches under alkaline conditions.

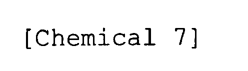

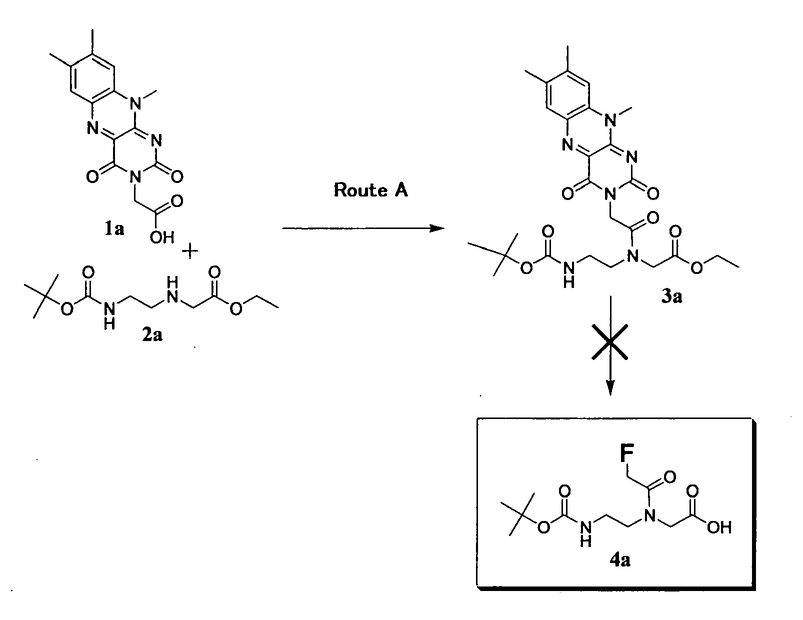

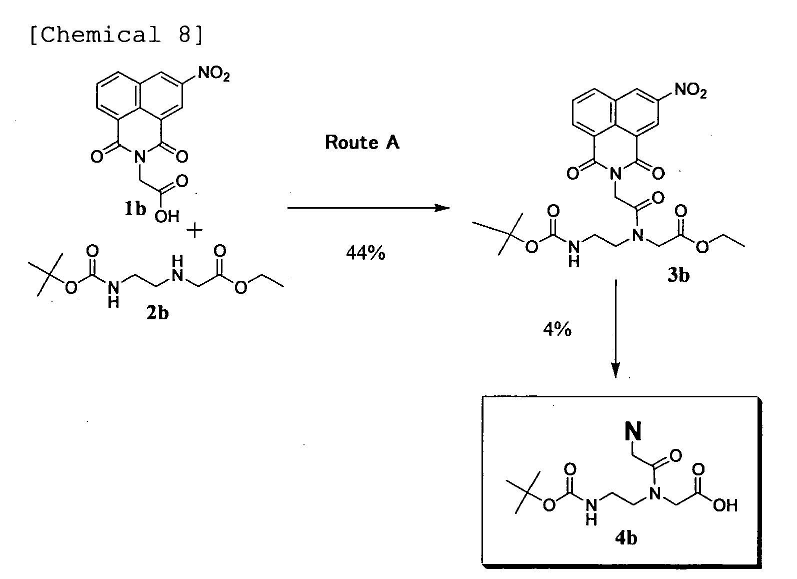

Thus, since there are typically many cases wherein functional molecules such as photofunctional molecules are expensive, methods for synthesizing more pertinent functional PNA, namely methods for extremely rapidly introducing these functional molecules for (1) efficient introduction of functional molecules into a PNA backbone structure in the design of functional PNA monomer units, (2) synthesis routes in consideration of cost performance, and (3) adaptation to applications as gene diagnostic drugs, have been sought.

In consideration of the above problems, the inventors of the present invention found a novel method for producing functional PNA monomers consisting of synthesizing a photofunctional PNA monomer 4 nearly quantitatively by using a t-butoxycarbonylaminoethylamine derivative 6 for the PNA backbone structure, and condensing with an active ester form 5 containing the pentafluorophenyl group of 1 as indicated in the following route B.

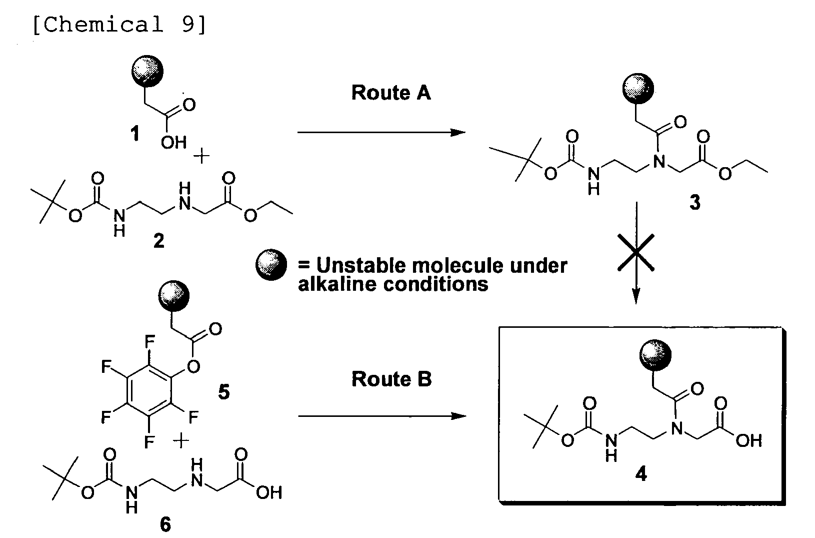

In addition, the inventors of the present invention found a different method for synthesizing functional PNA monomers by using a benzyloxycarbonyl-ω-amino acid derivative instead of the above t-butoxycarbonylaminoethylamine derivative 6 for the PNA backbone structure (route C). Patent applications have already been made for these methods.

Thus, methods for ultimately synthesizing functional PNA are being established industrially that consist of synthesizing functional PNA monomers according to methods using either of the above routes B or C, followed by polymerization of those monomers. Namely, it is becoming possible to industrially synthesize large volumes of functional PNA used as PNA probes using existing functional PNA synthesis methods.

On the other hand, improvements are also being made on methods for producing functional PNA for the purpose of improving cost performance and allowing ultra-high-speed introduction of functional molecules. For example, a method has been reported in which functional molecules are introduced into PNA oligomers post-synthetically by using the following precursor PNA monomer unit as a different approach from the method described above using functional PNA monomer units (Oliver Seitz; Tetrahedron Letters 1999, 40, 4161-4164).

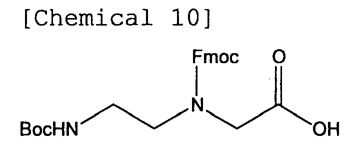

In this method, after introducing the above precursor PNA monomer unit into a PNA oligomer, functional PNA is synthesized by additionally introducing a functional molecule.

However, this method has the disadvantage of there being limitations on the types of functional molecules that can be introduced.

For example, as indicated below, the commercially available photofunctional molecule, succinimide ester, is unable to be introduced. Although it is necessary to first introduce a linker such as Fmoc-Gly in order to introduce this photofunctional molecule, the above compound becomes difficult to use as a result of this.

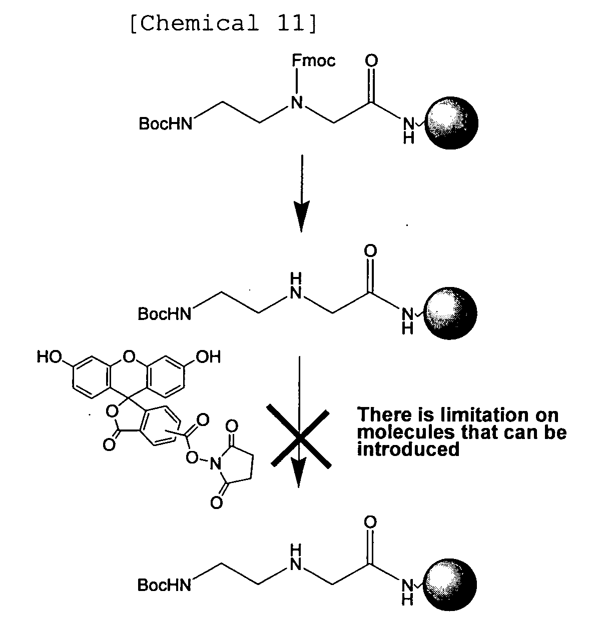

However, the inventors of the present invention surprisingly found that a functional PNA covering an extremely wide range can be synthesized that is capable of overcoming the aforementioned problems of the prior art, has superior cost performance and enables ultra-high-speed introduction of functional molecules by optimizing the structure of the precursor PNA monomer unit.

Namely, the inventors of the present invention succeeded in synthesizing a PNA oligomer by reacting a PNA monomer unit having adenine, guanine, cytosine or thymine protected by a protecting group with Fmoc-ω-amino acid-^{Boc} PNA-OH (IV) indicated below, followed by post-synthetically introducing a functional molecule having free carboxylic acid into said PNA oligomer: 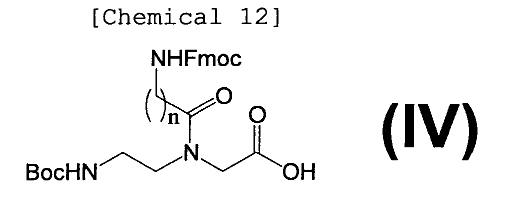 (wherein, n represents an integer of 1 to 15).

The aforementioned synthesis method enables a different type of functional molecule to be introduced after introducing a functional molecule. In addition, the introduced functional molecule is characterized by being selected from a photoreactive functional molecule, membrane-permeating functional molecule, organ-selective functional molecule, bactericidal functional molecule, molecule-destroying functional molecule, adhesive functional molecule or molecule-recognizing functional molecule.

In addition, since it is not necessary to use a commercially available succinimide ester as a functional molecule to be introduced, and since a compound can be used and quantitatively introduced without problem provided it is a compound having carboxylic acid, the aforementioned synthesis method has extremely superior cost performance.

Moreover, by dividing the resin after introducing the aforementioned precursor PNA monomer unit into the functional PNA oligomer, different functional molecules can be introduced for each resin, thereby making it possible to develop an extremely fast functional PNA oligomer synthesis method.

An example of a functional PNA oligomer that can be efficiently synthesized according to this method is a compound represented by the following general formula (V): 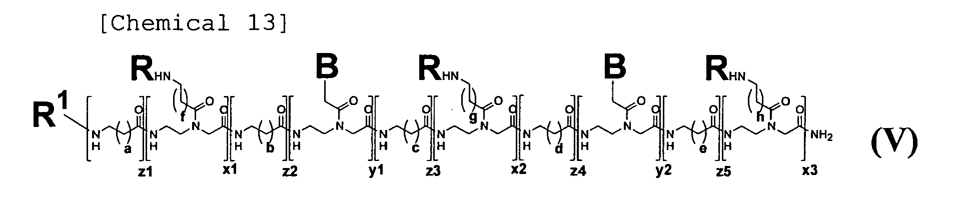 (wherein B's each independently represent adenine, guanine, cytosine or thymine, which may be the same or different, R's each independently represent an Fmoc group or a functional carboxylic acid derivative, which may be the same or different, R¹ represents a hydrogen atom or a functional carboxylic acid derivative, a through h represent an integer of 0 to 10, X₁ through X₃, Y₁, Y₂ and Z₁ through Z₅ all represent an integer of 0 or more, and Z₁ + Z₂ + Z₃ + Z₄ + Z₅ ≥ 0, provided that X₁ + X₂ + X₃ and Z₁ + Z₂ + Z₃ + Z₄ + Z₅ are not simultaneously 0, and in the case X₁ + X₂ + X₃ = 0, R¹ represents a functional carboxylic acid derivative), wherein Z₁ + Z₂ + Z₃ + Z₄ + Z₅ = 0 and R¹ represents a hydrogen atom.

However, although DNA oligomers, RNA oligomers and PNA oligomers have previously been used as fluorescent probes for introduction into cells, in order to introduce these into cells, they must naturally pass through the cell membrane. However, since the surface of the cell membrane is negatively charged, it is extremely difficult to introduce DNA/RNA oligomers which are inherently negatively charged.

On the other hand, although PNA oligomers are electrically neutral, this still results in difficulty in passing through the cell membrane. Thus, when introducing a PNA oligomer into a cell, the membrane surface is pretreated to facilitate introduction or the PNA oligomer is forcibly introduced by using a transfection reagent.

However, in the case of having introduced a PNA oligomer by carrying out such treatment, even if it demonstrates the function of a probe, there is no guarantee that it will necessarily accurately express behavior inherently exhibited by the body. Moreover, since this is the case for a single cell, it is all the more true for use among a large number of cells (individual body).

On the basis of the present circumstances and viewpoint, it is believed to be useful to develop a fluorescent PNA probe having a membrane permeability function.

It should be noted that fluorescent PNA probes having a membrane permeability function already exist. Examples of these include (1) that in which an oligopeptide having a membrane permeability function is bonded to PNA, and (2) that in which a phospholipid having a membrane permeability function is bonded to PNA. However, since those portions of these probes other than the PNA are decomposed by enzymes such as proteases within the cell after permeating the cell membrane, they are expected to remain inside the cell. Since excess PNA probe that has been unable to acquire a target loses its membrane permeability function and is difficult to move outside the cell in subsequent washing processes, this means that it is unable to accurately express the gene expression system inherently possessed by the cell.

However, the inventors of the present invention succeeded in designing a novel fluorescent PNA probe capable of accurately expressing a gene expression system without containing complex pretreatment or post-treatment, namely using live cells as such, by using a precursor PNA oligomer containing Fmoc-ω-amino acid-^{Boc}PNA-OH to post-synthetically introduce an amino acid derivative having a membrane permeability function.

However, this Fmoc-ω-amino acid-^{Boc}PNA-OH is not the only substance effective for this precursor PNA oligomer, but rather that role can also be fulfilled by a lysine derivative, which is an essential amino acid, and the design of novel fluorescent PNA probes that take into consideration ease of handling and ease of acquisition are predicted to become necessary in the future.

Thus, the object of the present invention is to provide a novel synthesis method of functional PNA, a compound used therein, and a novel functional PNA, that has superior cost performance and enables ultra-high-speed introduction of functional molecules.

### DISCLOSURE OF THE INVENTION

As a result of extensive studies in consideration of the aforementioned problems, the inventors of the present invention surprisingly found that a functional PNA can be synthesized that overcomes the aforementioned problems of the prior art and covers an extremely wide range by optimizing the structure of the precursor PNA monomer unit, thereby leading to completion of the present invention as described below.
(1) A method for producing a functional PNA oligomer comprising: synthesizing a PNA oligomer by reacting a PNA monomer unit having adenine, guanine, cytosine or thymine protected by a protecting group with Boc-lysine(Fmoc)-OH according to general formula (I) (wherein Fmoc represents 9-fluorenylmethoxycarbonyl) or Fmoc-lysine(Alloc)-OH according to general formula (II) (wherein Fmoc represents 9-fluorenylmethoxycarbonyl, Boc represents t-butoxycarbonyl, and Alloc represents allyloxycarbonyl), followed by introducing a functional molecule having a free carboxylic acid into said PNA oligomer and de-protecting the protecting group.
(2) The method for producing a functional PNA oligomer according to (1) above, wherein a different type of functional molecule is introduced after having introduced a functional molecule.
(3) The method according to (1) above, wherein the functional molecule that is introduced is selected from a photoreactive functional molecule, membrane-permeating functional molecule, organ-selective functional molecule, bactericidal functional molecule, molecule-destroying functional molecule, adhesive functional molecule or molecule-recognizing functional molecule.
(4) The method according to (2) or (3) above, wherein the functional molecule that is introduced contains a photofunctional molecule and a membrane-permeable functional molecule.
(5) The method according to (4) above, wherein the photofunctional molecule is the following Cy3, Cy5, Bodipy, pyrene, naphthalimide, naphthaldiimide, FAM, FITC, ROX, TAMRA or Dabcyl, and the membrane-permeable functional molecule is a water-soluble amino acid derivative. 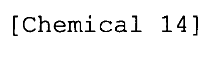 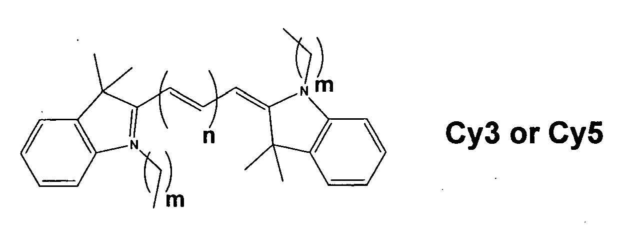 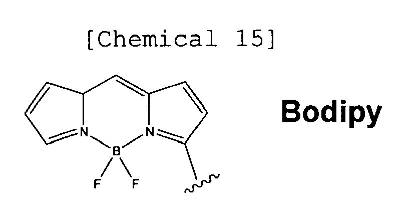 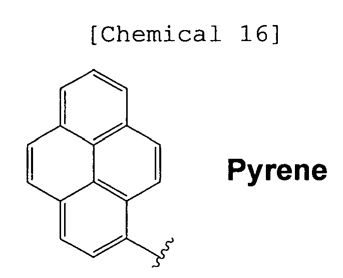 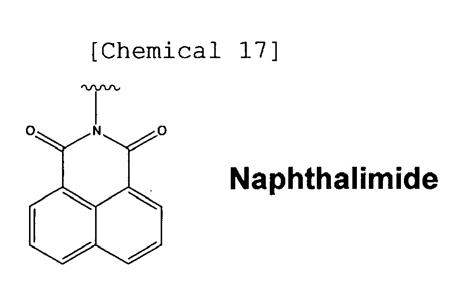 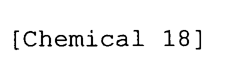 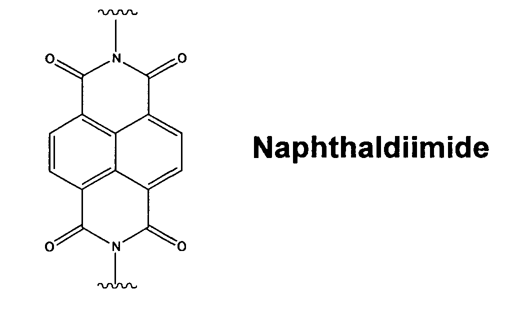 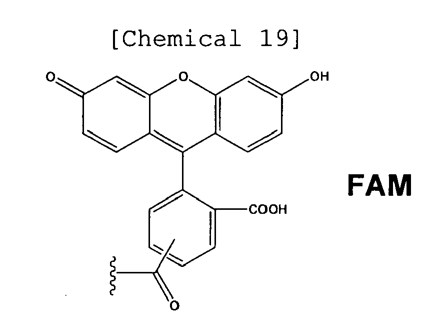 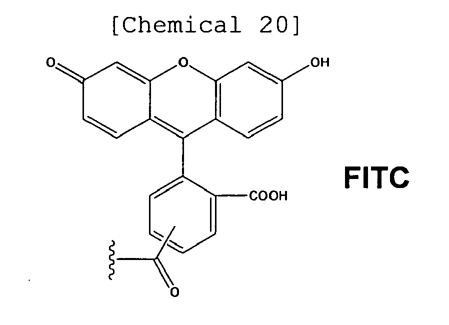 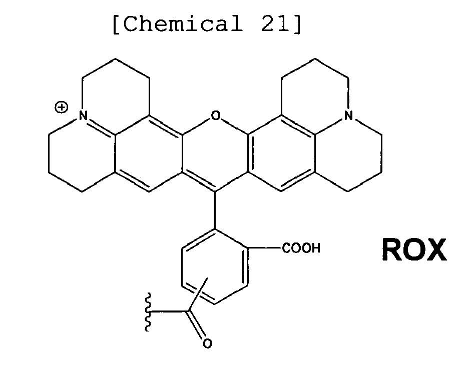 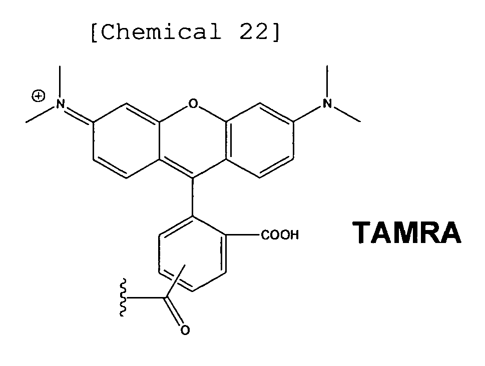 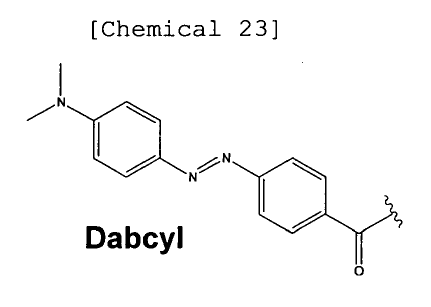
(6) The method according to any of (1) to (5) above, wherein the protecting group that protects adenine, guanine, cytosine or thymine is a benzyloxycarbonyl group (Z group).
(7) The method according to any of (1) to (6) above, wherein synthesis of PNA oligomer includes condensation and elongation in the PNA chain using solid-phase supports for the Boc method and Fmoc method.
(8) The method according to any of (1) to (7) above, wherein the solid-phase support for the Boc method is methylbenzhydrylamine (MBHA) resin used for peptide synthesis with the solid-phase Boc method.
(9) The method according to any of (1) to (7) above, wherein the solid-phase support for the Fmoc method is MBHA, a resin in which polystyrene is chloromethylated (Merrifield resin), a Merrifield resin modified with 4-hydroxybenzyl alcohol (Wang resin), an aminomethyl resin bonded with a Boc-amino acid linker (PAM resin), an aminomethyl resin bonded with an N-Fmoc-N-methoxy linker (Weinreb resin), a resin in which p-nitrobenzophenonoxime is bonded to polystyrene (Oxime resin) or a resin that has been tritylated using polystyrene (Trityl resin).
(10) The method according to any of (1) to (9) above, wherein the introduction of a functional molecular having free carboxylic acid is carried out by dehydration condensation with a primary amino group obtained by selective de-protection by piperidine treatment of an Fmoc group in the Boc method or by zinc acetate solution treatment of an Alloc group in the Fmoc method.
(11) The method according to (2) above comprising the following a) to d),
   one or two or more of a) and b):
   a) production of a PNA oligomer by reacting a PNA monomer unit with Boc-lysine(Fmoc)-OH in a step of introducing Boc-lysine(Fmoc)- OH into a PNA oligomer;
   b) introduction of a functional molecule into a PNA oligomer is carried out by dehydration condensation with a primary amino group obtained by selective de-protection by piperidine treatment of an Fmoc group in the aforementioned step of producing a functional PNA oligomer from a PNA oligomer; and

   one or two or more of c) and d):
   c) production of a PNA oligomer by reacting a PNA monomer unit with Fmoc-lysine(Alloc)-OH in a step of introducing Fmoc-lysine (Alloc)-OH into a PNA oligomer; and,
   d) introduction of a functional molecule into a PNA oligomer is carried out by dehydration condensation with a primary amino group obtained by selective de-protection by zinc acetate solution treatment of an Alloc group in the aforementioned step of producing a functional PNA oligomer from a PNA oligomer.
(12) A compound represented by the following general formula (III): 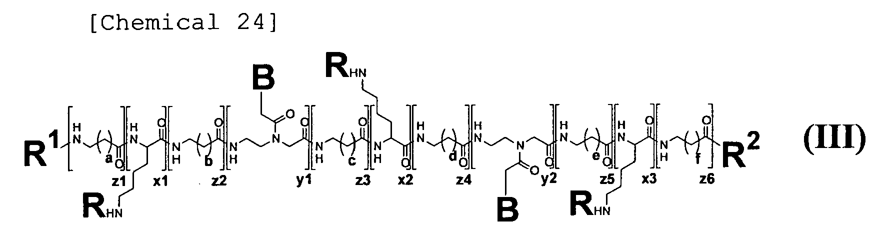 (wherein B's each independently represent adenine, guanine, cytosine or thymine, which may be the same or different, R's each independently represent an Fmoc group or a functional carboxylic acid derivative, R¹ represents a hydrogen atom or a functional carboxylic acid derivative, R² represents a derivative or a functional carboxylic acid derivative containing a hydrogen atom, an amino group, a hydroxyl group or a thiol group, a through f represent an integer from 0 to ∞, X₁ through X₃, Y₁, Y₂ and Z₁ through Z₆ all represent an integer of 0 or more, X₁ + X₂ + X₃ ≥ 0, Y₁ + Y₂ > 0 and Z₁ + Z₂ + Z₃ + Z₄ + Z₅ ≥ 0, provided that X₁ + X₂ + X₃ and Z₁ + Z₂ + Z₃ + Z₄ + Z₅ are not simultaneously 0, and in the case X₁ + X₂ + X₃ = 0, R¹ represents a functional carboxylic acid derivative).
(13) The compound according to (12) above, wherein X₁ + X₂ + X₃ = 3 and Y₁ + Y₂ = 15.
(14) The compound according to (13) above, wherein X₁ = 3 and Y₁ = 15.
(15) The compound according to (14) above, wherein R or R¹ represents a cell membrane-permeable functional molecule derivative.
(16) The compound according to (15) above, wherein R¹ represents a functional carboxylic acid derivative.
(17) The compound according to (15) or (16) above, wherein X₁ = Z₁ = 1.
(18) The compound according to any of (15) to (17), wherein Y₁ ≥ 2 and Z₂ = 1.
(19) The compound according to any of (15) to (18), wherein a ≤ 6, b ≤ 4 and f ≤ 6.
(20) The compound according to any of (15) to (19), wherein R¹ represents a photofunctional carboxylic acid derivative.

The present invention succeeds in being able to synthesize a photofunctional PNA oligomer nearly quantitatively by post-synthetically introducing a functional molecule after having introduced Boc-lysine(Fmoc)-OH or Fmoc-lysine(Alloc)-OH into a PNA oligomer.

Furthermore, each of the gists of Fmoc, Boc and Alloc are as indicated in (1) above that explains the evidence.

According to the aforementioned characteristic, in a production method of the present invention, it is not necessary to use a commercially available succinimide ester as a functional molecule to be introduced, and any compound can be used and quantitatively introduced without problem provided it is a compound having carboxylic acid.Consequently, a production method according to the present invention has extremely superior cost performance.

In addition, by dividing the resin after introducing the aforementioned precursor PNA monomer unit into the functional PNA oligomer, different functional molecules can be introduced for each resin. Thus, according to a production method of the present invention, an extremely fast functional PNA oligomer synthesis method can be developed.

An example of a functional PNA oligomer that can be efficiently synthesized according to the method of the present invention is a compound represented by the following general formula (III): 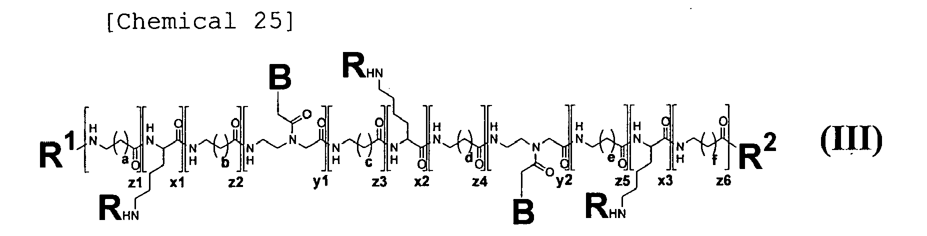 (wherein B's each independently represent adenine, guanine, cytosine or thymine, which may be the same or different, R's each independently represent an Fmoc group or a functional carboxylic acid derivative, which may be the same or different, R¹ represents a hydrogen atom or a functional carboxylic acid derivative, R² represents a derivative or a functional carboxylic acid derivative containing a hydrogen atom, an amino group, a hydroxyl group or a thiol group, a through f represent an integer from 0 to ∞, X₁ through X₃, Y₁, Y₂ and Z₁ through Z₆ all represent an integer of 0 or more, X₁ + X₂ + X₃ ≥ 0, Y₁ + Y₂ > 0 and Z₁ + Z₂ + Z₃ + Z₄ + Z₅ ≥ 0, provided that X₁ + X₂ + X₃ and Z₁ + Z₂ + Z₃ + Z₄ + Z₅ are not simultaneously 0, and in the case X₁ + X₂ + X₃ = 0, R¹ represents a functional carboxylic acid derivative), wherein Z₁ + Z₂ + Z₃ + Z₄ + Z₅ = 0 and R¹ represents a hydrogen atom.

According to the present invention, the same or different functional molecule can be introduced at a plurality of arbitrary locations in a compound represented by the aforementioned general formula (I). Namely, although this is the result of collectively carrying out piperidine treatment or zinc acetate solution treatment and post-synthetic introduction of a functional molecule after having introduced a PNA oligomer using the aforementioned precursor PNA monomer units, this is essential for rapidly designing antennapedia (group of genes in which leg part is formed at the joint of an antenna or a feeler) that improves the cell membrane permeation function of the PNA oligomer. A method according to the present invention is extremely superior with respect to this point as well.

An example of a compound produced in this manner is that in which Z₁ + Z₂ + Z₃ + Z₄ + Z₅ > 0, and R represents a cell membrane permeable molecule derivative, and R¹ represents a functional carboxylic acid derivative in the aforementioned general formula (I).

This probe can be broadly divided into a fluorescent labeled region, a cell membrane permeability function region, and a molecule-recognizing region, and has a form in which each is bonded through a linker site (portions represented by the suffixes Z₁ through Z₅).

Commercially available products and novel fluorescent labeled PNA monomer units that have been previously filed for PCT application by the inventors of the present invention can be used for the fluorescent labeled compound.

The molecule-recognizing site is synthesized using commercially available PNA units. These are characterized by the use of Boc-lysine(Fmoc)-OH or Fmoc-lysine(Alloc)-OH for the membrane permeability function region as precursor units for post-synthetically introducing a functional molecule. Said precursor units are commercially available, and are characterized by allowing the same functional molecules to be collectively introduced as previously described after introducing a plurality of these in a row.

Thus, according to the present invention, various functional molecules can be easily and extremely efficiently introduced into PNA without any limitations on the photofunctional molecule.

Examples of these functional molecules include photofunctional monomer units such as Cy3, Cy5, Bodipy, naphthalimide, naphthaldiimide, flavin, Dabcyl, biotin, FAM, rhodamine, TAMRA, ROX, HABA, pyrene and coumarine types, membrane-permeable functional molecules, organ-selective functional molecules, bactericidal functional molecules, molecule-destroying functional molecules, adhesive functional molecules and molecule-recognizing functional molecules.

Namely, the term "functional" in the present invention refers not only to photofunctionality, but also to all of the various functions newly imparted to compounds by carrying out a specific modification, including membrane permeability, organ selectivity, bactericidal activity, molecule destruction, adhesiveness and molecule recognition.

Moreover, the term "functional PNA" in the present invention refers not only to the direct bonding of corresponding PNA monomers according to a 2-(N-aminoethyl)glycine skeleton, but also includes a precursor lysine skeleton in which a hydrocarbon chain and a functional molecule are introduced therebetween.

### BRIEF DESCRIPTION OF THE DRAWINGS

Fig. 1 is a drawing representing differences in the structure and charge status of DNA and PNA.
Fig. 2 is a drawing representing the structures of two types of PNA monomer units.

The characteristic of the method of the present invention is explained in more detail here.

### BEST MODE FOR CARRYING OUT THE INVENTION

Typical routes for synthesizing an oligo-PNA according to the present invention are as shown in Chemical 26 and Chemical 27 below.

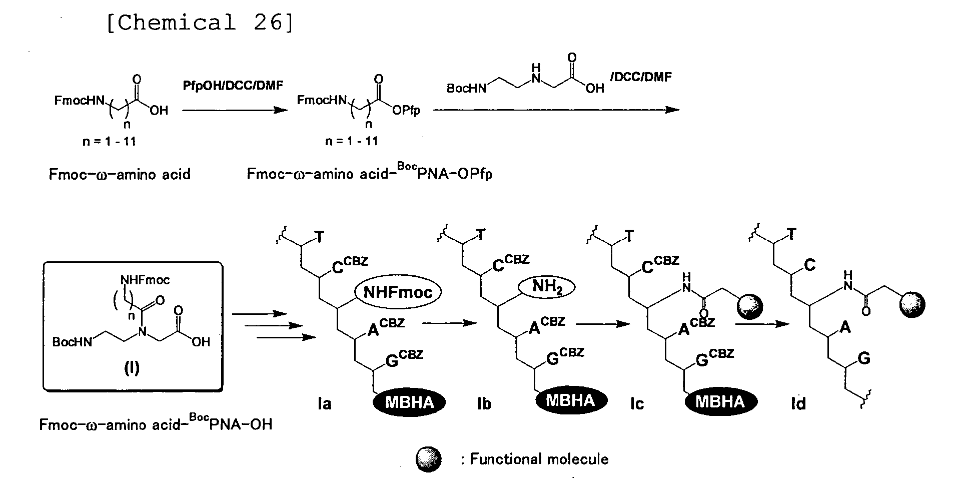

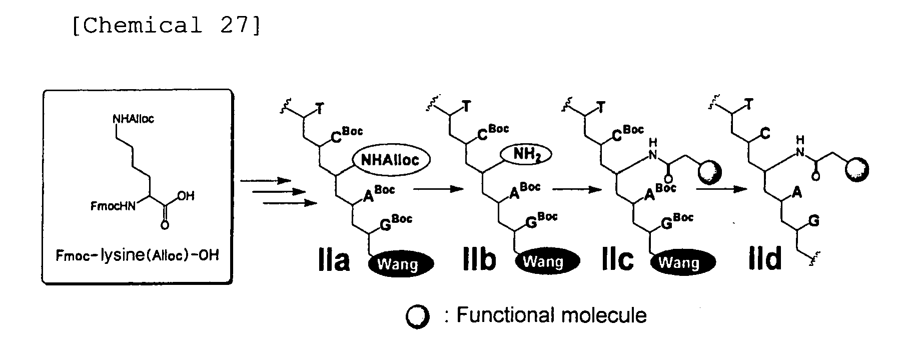

Furthermore, MBHA refers to a methylbenzhydrylamine resin used for peptide synthesis with solid-phase Boc, while Wang refers to a Merrifield resin modified with 4-hydroxybenzyl alcohol, and these are as described in [0036] and [0037].

Next, in the production step relating to [Chemical 26], oligomer Ia is synthesized using Boc-lysine(Fmoc)-OH as shown in [Chemical 28] below. 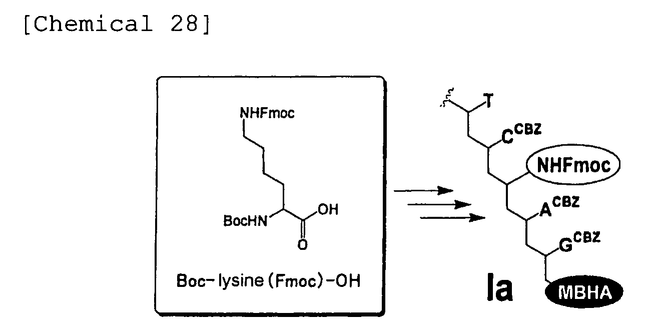
More specifically, a PNA monomer unit having adenine, guanine, cytosine or thymine protected with a Z group (N-benzyloxycarbonyl group) and so forth is reacted with a precursor PNA monomer unit and the PNA chain is successively condensed and elongated using a Boc solid-phase support.

In condensing the PNA chain, although it is necessary to eliminate the Boc group in advance, there are no limitations on the method of accomplishing this, and an ordinary method is used. A typical condensation agent such as HATU, HBTU or BOP is used for the subsequent condensation.

In addition, although there are no particular limitations on the solid-phase support provided it is that which is used for the Boc method, MBHA is used particularly preferably.

Next, in the production step relating to [Chemical 26], Ib is obtained by selectively de-protecting the Fmoc group by piperidine treatment to obtain an amino group as shown in the following [Chemical 29], 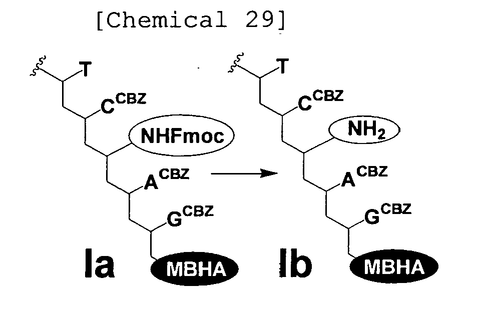
after which Ic is obtained by dehydrating and condensing a functional molecule having a free carboxylic acid to the aforementioned amino group of said Ib as shown in the following [Chemical 30].

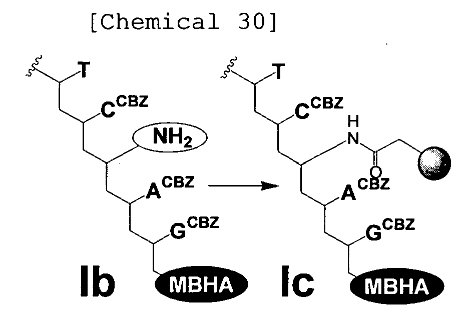

Although there are no particular limitations on the aforementioned carboxylic acid, the use of an aliphatic carboxylic acid is preferable since it increases production efficiency since aliphatic carboxylic acids exceed aromatic carboxylic acids in terms of reactivity.

In addition, de-protection of the Fmoc group by piperidine treatment is preferably carried out by allowing a certain amount of time. A period of 20 to 40 minutes is particularly preferable, while a period of 30 minutes is the most preferable.

There are no particular limitations on the type of condensation agent, and a typical condensation agent such as HATU, HBTU or BOP is used similar to the aforementioned condensation of the PNA chain.

Furthermore, introduction of the functional molecule may be carried out immediately after a Boc-lysine(Fmoc)-OH has been condensed, or after all PNA monomer units containing Boc-lysine(Fmoc)-OH have been successively condensed.

Finally, in the production step relating to [Chemical 26], the desired PNA oligomer Id is obtained by simultaneously carrying out severing from the support resin and de-protection of the Z group as shown in the following [Chemical 31]

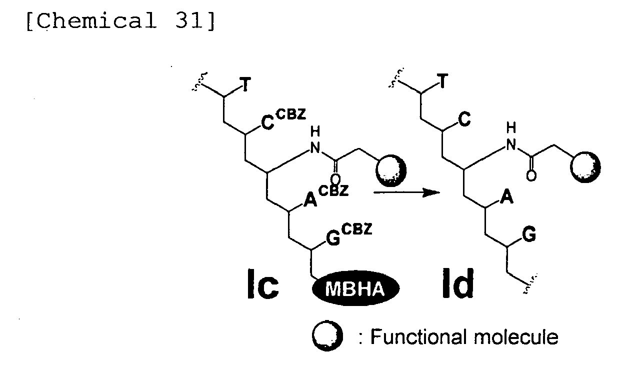

There are no particular limitations on the conditions for severing and de-protection provided they are carried out after de-protection of the Fmoc group. For example, severing and de-protection are preferably carried out under ordinary conditions such as TFA/TFMSA/p-cresol/thioanisole = 60/25/10/10.

Next, in the production step relating to [Chemical 27], oligomer IIa is synthesized using Fmoc-lysine(Alloc)-OH as shown in the following [Chemical 32]. 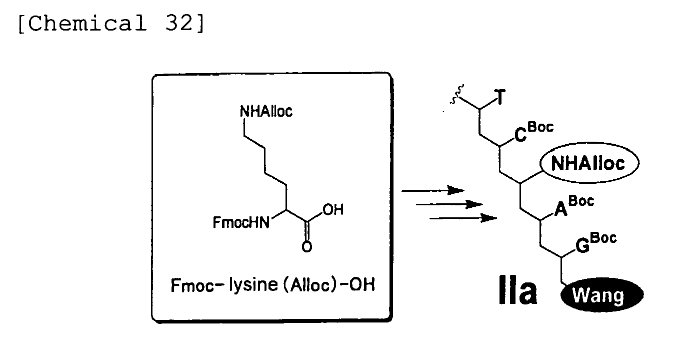 More specifically, a PNA monomer unit having adenine, guanine, cytosine or thymine protected with a Boc group and so forth is reacted with a precursor PNA monomer unit followed by successive condensation and elongation of the PNA chain using an Fmoc solid-phase support.

In condensing the PNA chain, although it is necessary to eliminate the Fmoc group in advance, there are no limitations on the method of accomplishing this, and an ordinary method is used. A typical condensation agent such as HATU, HBTU or BOP is used for the subsequent condensation.

In addition, although there are no particular limitations on the solid-phase support provided it is that which is used for the Fmoc method, Wang is used particularly preferably.

Next, in the production step relating to [Chemical 27], IIb is obtained by selectively de-protecting the Fmoc group by zinc acetate solution treatment to obtain an amino group as shown in the following [Chemical 33], 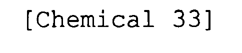 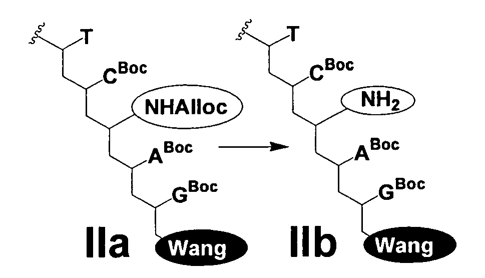 after which IIc is obtained by dehydrating and condensing a functional molecule having a free carboxylic acid to the aforementioned amino group of said IIb as shown in the following [Chemical 34]

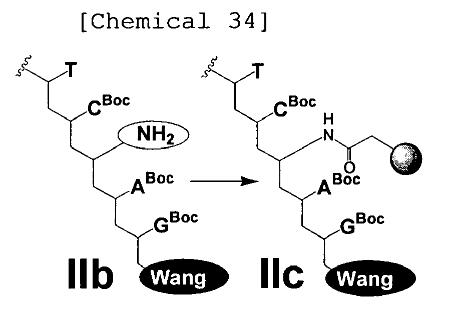

Although there are no particular limitations on the aforementioned carboxylic acid, the use of an aliphatic carboxylic acid is preferable due to the fact that it increases production efficiency since aliphatic carboxylic acids exceed aromatic carboxylic acids in terms of reactivity.

In addition, de-protection of the Alloc group by zinc acetate solution treatment is preferably carried out by allowing a certain amount of time. A period of 10 minutes to 1 hour is preferable.

There are no particular limitations on the type of condensation agent, and a typical condensation agent such as HATU, HBTU or BOP is used similar to the aforementioned condensation of the PNA chain.

Furthermore, introduction of the functional molecule may be carried out immediately after a Fmoc-lysine(Alloc)-OH has been condensed, or after all PNA monomer units containing Fmoc-lysine(Alloc)-OH have been successively condensed.

Finally, in the production step relating to [Chemical 27], the desired PNA oligomer IId is obtained by simultaneously carrying out severing from the support resin and de-protection of the Boc group as shown in the following [Chemical 35].

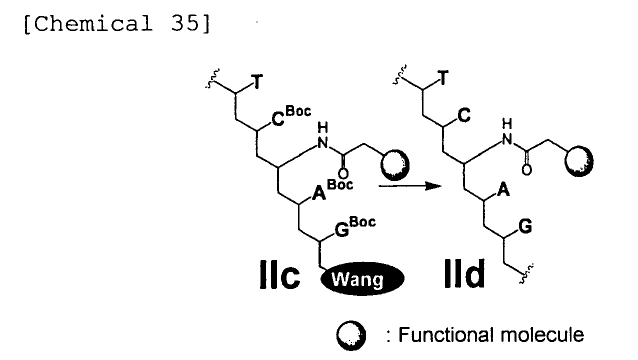

There are no particular limitations on the conditions for severing and de-protection provided they are carried out after de-protection of the Fmoc group. For example, severing and de-protection are preferably carried out under ordinary conditions such as TFA/p-cresol = 95/5.

As has been described above, in a method according to the present invention, differing from methods requiring synthesis of an active ester used in functional monomer synthesis of the prior art, the functional molecule can be used as such. In addition, since various functional molecules can be introduced once IIa has been synthesized, the rapid and parallel synthesis of various PNA probes, which was difficult in the prior art, now becomes possible.

According to a method of the present invention that contains the reaction between Boc-lysine(Fmoc)-OH or Fmoc-lysine(Alloc)-OH and a molecule having a PNA chain, a compound represented by the following general formula (III) : 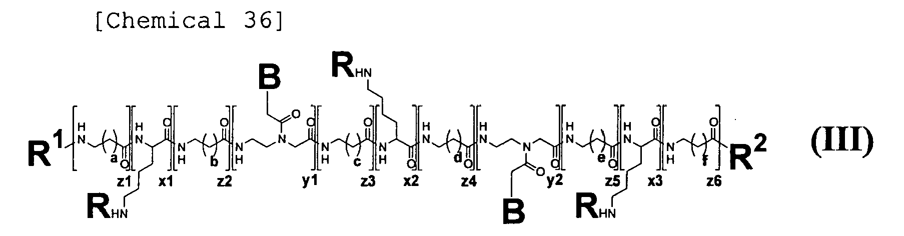 (wherein B's each independently represent adenine, guanine, cytosine or thymine, which may be the same or different, R's each independently represent an Fmoc group or a functional carboxylic acid derivative, which may be the same or different, R¹ represents a hydrogen atom or a functional carboxylic acid derivative, R² represents a derivative or a functional carboxylic acid derivative containing a hydrogen atom, an amino group, a hydroxyl group or a thiol group, a through f represent an integer from 0 to ∞, X₁ through X₃, Y₁, Y₂ and Z₁ through Z₆ all represent an integer of 0 or more, X₁ + X₂ + X₃ ≥ 0, Y₁ + Y₂ > 0 and Z₁ + Z₂ + Z₃ + Z₄ + Z₅ ≥ 0, provided that X₁ + X₂ + X₃ and Z₁ + Z₂ + Z₃ + Z₄ + Z₅ are not simultaneously 0, and in the case X₁ + X₂ + X₃ = 0, R¹ represents a functional carboxylic acid derivative), wherein Z₁ + Z₂ + Z₃ + Z₄ + Z₅ = 0 and R¹ represents a hydrogen atom, can be exemplified.

In addition, in a compound represented by the aforementioned general formula (III), a compound in which, for example, R or R¹ represents a cell membrane-permeable functional molecule derivative is preferably synthesized as a compound in which a plurality of functional molecules are introduced. A typical example of such a compound is that in which R represents a cell membrane-permeable function molecule derivative and R¹ represents a functional carboxylic acid derivative such as a photofunctional molecule, namely a compound in which functional molecules are introduced at a plurality of sites including the terminal section, and a plurality of functions are imparted thereby. A schematic representation of an example of such a compound is shown below.

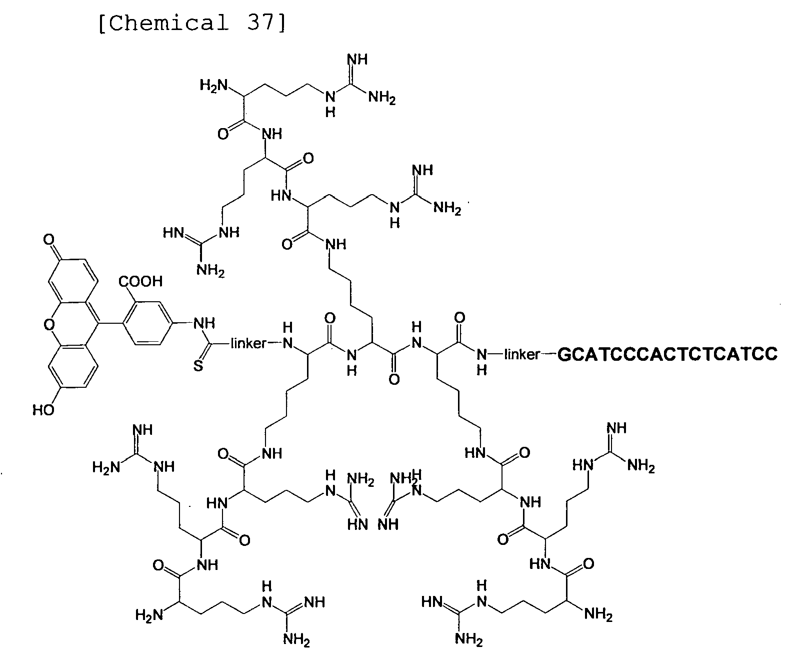

Such a compound is a compound in which, for example, X₁ = Z₁ = Z₂ = 1 and Y₁≥ 2 in the aforementioned general formula (III). Such a compound is preferable in terms of ease of synthesis, synthesis cost and so forth.

Although there are no particular limitations on the aforementioned compound provided a, b and f are each an integer of 0 to 10, even if, for example, a ≤ 6, b ≤ 4 and f ≤ 6, there are no particular problems in terms of synthesis or practical use.

The introduction of linker sites prevents interference between individual functional sites and base sequence recognition regions, and enables the function of the molecule to be demonstrated more reliably. The terms PNA, PNA monomer and PNA oligomer in the present specification include those containing linker sites in their terminals and/or internally.

In addition to the aforementioned linker sites, mutual interference between these sites or regions can also be prevented by selecting f through h in general formula (III) as desired.

Although examples of groups that compose the linker sites include linear or branched hydrocarbons and their ether forms, linear hydrocarbon groups are preferable in terms of ease of introduction and cost, and linear hydrocarbon groups having 1 to 6 carbon atoms are particularly preferable. The ether forms are preferable in terms of their general applicability.

The aforementioned compounds in which a plurality of functional molecules have been introduced are preferably synthesized using, for example, the method of Koch, T.; Hansen, H.F.; Andersen, P.; Larsen, T.; Batz, H.G.; Otteson, K.; Orum, H.: *J. Peptide Res.* 1997, 49, 80-88.

A base sequence recognition site can be converted to an oligomer by solid-phase synthesis using various types of commercially available PNA monomers. Commercially available Boc-7-aminoheptanoic acid or Boc-6-aminocaproic acid and so forth can be used for linker sites.

If a photofunctional molecule is introduced as a functional molecule of general formula (III), the molecule can be fluorescent-labeled and compounds can be synthesized having other functions as well. Although various fluorescent emission wavelengths can be selected using commercially available active ester-type fluorescent labeled compounds such as commercially available Cy3, Cy5, Bodipy, pyrene, naphthalimide, naphthaldiimide, FAM, FITC, ROX, TAMRA and Dabcyl for this type of fluorescent labeling site, the fluorescent-labeled compound that is introduced is not limited to these.

An example of another function that can be introduced into a compound of the present invention includes a membrane permeability function. This type of membrane permeability function site can be similarly introduced by using a compound represented by the aforementioned general formula (III) of the previous patent. While arginine is an example of a functional molecule that is able to improve membrane permeability, water-soluble amino acids such as lysine and serine can also be used preferably.

In addition, a plurality of amino acids can also be introduced by using Boc-lysine(Fmoc)-OH or Fmoc-lysine (Alloc)-OH. An example of this synthesis is shown in Example 1. However, the aforementioned compounds are only model compounds of a fluorescent PNA probe having a membrane permeability function according to the present invention, and the present invention is not limited thereto.

These probes are characterized by "having complete enzyme resistance since they are all of the PNA type". Namely, although previous probes having a membrane permeability function consisted primarily of those in which the PNA and peptide chain or phospholipid having the membrane permeability function were covalently bonded, and although these known probes have a superior membrane permeability function, once they enter the cell, the peptide chain or phospholipid is expected to be decomposed by enzyme groups. Thus, these have the disadvantage in that a decomposed probe that is not recognized as a target cannot be completely removed in the washing process.

In contrast, the probe designed here enables accurate quantitative determination of the amount of expressed gene since probe that does not recognize the target is able to be completely removed in the washing process as a result of not being subjected to enzyme decomposition within the cell.

Furthermore, in addition to compounds having these functions, organ-selective functional molecules such as lactose and tris-X, bactericidal functional molecules such as tanatin and secropin, molecule-recognizing functional molecules such as viologen, and molecule-destroying functional molecules such as N-methylhydroxamic acid can also be introduced without limitation according to the present invention, and such compounds can be provided for practical use at considerably lower cost.

### Examples

Although the following provides a more detailed explanation of the present invention using its examples, the scope of the present invention is not limited to these examples.

### Example 1 - Synthesis of Fluorescent PNA Probe Having Membrane Permeability Function

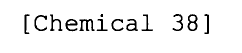

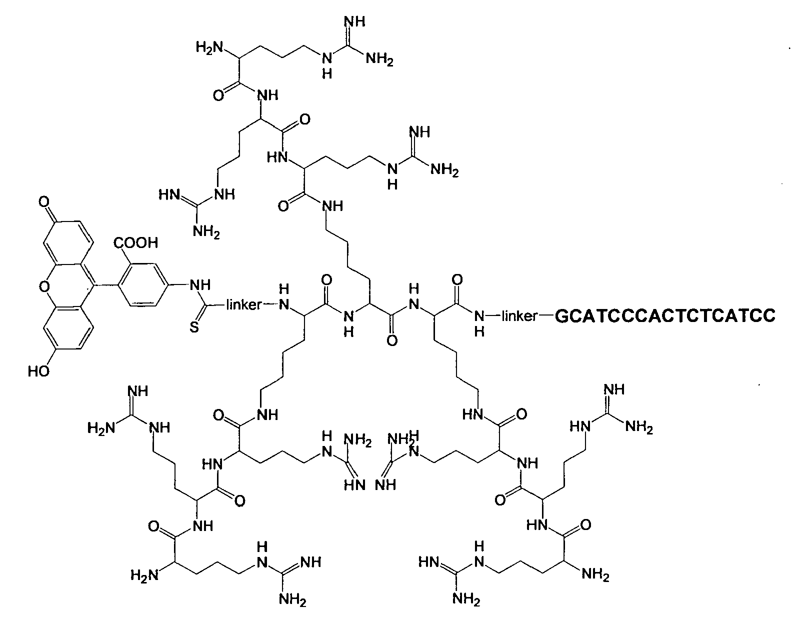

A sequential elongation reaction was first carried out on solid-phase support MBHA (50 mg) using a thymine PNA monomer unit (7.7 mg, 20 mmol), a condensation agent HBTU (7.6 mg, 20 mmol) and DIEA (3.5 mL, 20 mmol) in accordance with the standard Boc method (cf. Koch, T.; Hansen, H.F.; Andersen, P.; Larsen, T.; Batz, H.G.; Otteson, K.; Orum, H.: *J. Peptide Res.* 1997, 49, 80-88) (design of base sequence recognition region).

Next, linker ω-amino acid-Boc-7-aminoheptanoic acid (5.2 mg, 20 mmol), Fmoc-Ahx-^{Boc} PNA-OH (10.0 mg, 20 mmol) and Boc-7-aminoheptanoic acid again were sequentially condensed using a condensation agent HBTU (7.6 mg, 20 mmol) and DIEA (3.5 mg, 20 mmol) (design of linker site and membrane permeability function region).

After all units had been sequentially condensed, the Fmoc group was de-protected by piperidine treatment (20% piperidine in DMF, room temperature for 3 minutes). Next, a functional carboxylic acid derivative in the form of naturally-occurring Fmoc-Arg(Mts)-OH (23.1 mg, 40 mmol) was condensed using a condensation agent HBTU (15.2 mg, 40 mmol) and DIEA (7.0 mL, 40 mmol) to introduce the functional molecule at the desired site (introduction of membrane permeability function).

After de-protecting the Fmoc group by piperidine treatment (20% piperidine in DMF, room temperature for 3 minutes), naturally-occurring Fmoc-Arg(Mts)-OH (23.1 mg, 40 mmol) was condensed again using a condensation agent HBTU (15.2 mg, 40 mmol) and DIEA (7.0 mL, 40 mmol) to introduce a functional molecule at the desired site. This was then repeated one more time (additional introduction of membrane permeability function).

After de-protecting the Boc group by TFA treatment (95% TFA/5% m-cresol), FITC (9.3 mg, 25 mg) was fluorescent-labeled by stirring at room temperature for 12 hours in the presence of DIEA (17.4 mg, 100 mmol) (design of fluorescent-labeled site).

Finally, after de-protecting the remaining Fmoc group by piperidine treatment (2-piperidinein D, room temperature for 3 minutes), the fluorescent PNA probe was severed from the solid-phase support according to ordinary methods (TFA/TFMSA/p-cresol/thioanisol = 60/25/10/10) followed by post-treatment to obtain the desired product. MALDI-TOF
MS: calcd. 6373.0231 (M+H⁺).

### Example 2 - Synthesis of Fluorescent PNA Probe Having Membrane Permeability Function

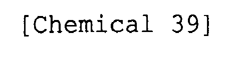

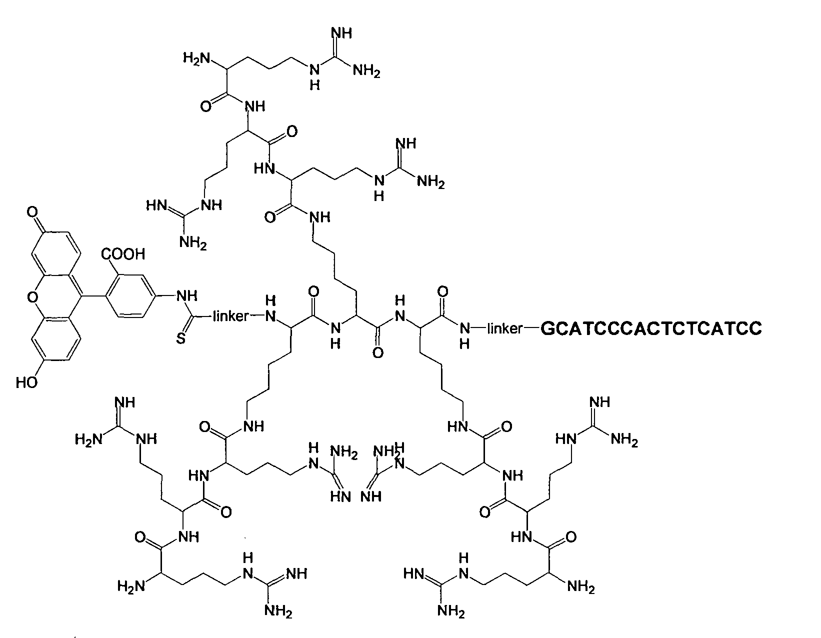

A sequential elongation reaction was first carried out on solid-phase support MBHA (50 mg) using a thymine PNA monomer unit (7.7 mg, 20 mmol), a condensation agent HBTU (7.6 mg, 20 mmol) and DIEA (3.5 mL, 20 mmol) in accordance with the standard Boc method (cf. Koch, T.; Hansen, H.F.; Andersen, P.; Larsen, T.; Batz, H.G.; Otteson, K.; Orum, H.: *J. Peptide Res.* 1997, 49, 80-88) (design of base sequence recognition region).

Next, linker ω-amino acid-Boc-7-aminoheptanoic acid (5.2 mg, 20 mmol), Fmoc-Ahx-^{Boc}PNA-OH (10.0 mg, 20 mmol) and Boc-7-aminoheptanoic acid again were sequentially condensed using a condensation agent HBTU (7.6 mg, 20 mmol) and DIEA (3.5 mg, 20 mmol) (design of linker site and membrane permeability function region).

After all units had been sequentially condensed, the Fmoc group was de-protected by piperidine treatment (20% piperidine in DMF, room temperature for 3 minutes). Next, a functional carboxylic acid derivative in the form of naturally-occurring Fmoc-Arg(Mts)-OH (23.1 mg, 40 mmol) was condensed using a condensation agent HBTU (15.2 mg, 40 mmol) and DIEA (7.0 mL, 40 mmol) to introduce the functional molecule at the desired site (introduction of membrane permeability function).

After de-protecting the Fmoc group by piperidine treatment (20% piperidine in DMF, room temperature for 3 minutes), naturally-occurring Fmoc-Arg(Mts)-OH (23.1 mg, 40 mmol) was condensed again using a condensation agent HBTU (15.2 mg, 40 mmol) and DIEA (7.0 mL, 40 mmol) to introduce a functional molecule at the desired site. This was then repeated one more time (additional introduction of membrane permeability function).

After de-protecting the Boc group by TFA treatment (95% TFA/5% m-cresol), FITC (9.3 mg, 25 mg) was fluorescent-labeled by stirring at room temperature for 12 hours in the presence of DIEA (17.4 mg, 100 mmol) (design of fluorescent-labeled site).

Finally, after de-protecting the remaining Fmoc group by piperidine treatment (2-piperidinein D, room temperature for 3 minutes), the fluorescent PNA probe was severed from the solid-phase support according to ordinary methods (TFA/TFMSA/p-cresol/thioanisol = 60/25/10/10) followed by post-treatment to obtain the target product. MALDI-TOF MS: calcd. 6373.0231 (M+H⁺).

### INDUSTRIAL APPLICABILITY

According to the present invention, since various functional molecules, without limiting to photofunctional molecules, can be easily and efficiently introduced into PNA making it possible to construct various PNA's used in gene therapy and the like, the present invention can be used in a wide range of fields of medical and pharmaceutical industries.

## Claims

1. A method for producing a functional PNA oligomer comprising: synthesizing a PNA oligomer by reacting a PNA monomer unit having adenine, guanine, cytosine or thymine protected by a protecting group with Boc-lysine(Fmoc)-OH according to general formula (I) (wherein Fmoc represents 9-fluorenylmethoxycarbonyl) or Fmoc-lysine(Alloc)-OH according to general formula (II) (wherein Fmoc represents 9-fluorenylmethoxycarbonyl, Boc represents t-butoxycarbonyl, and Alloc represents allyloxycarbonyl), followed by introducing a functional molecule having a free carboxylic acid into said PNA oligomer and de-protecting the protecting group.

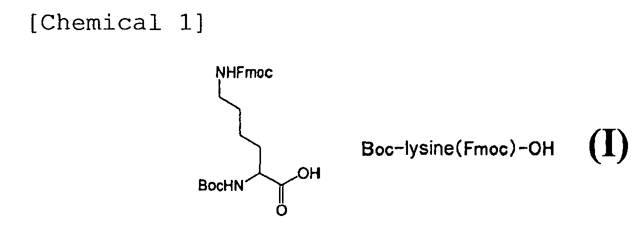

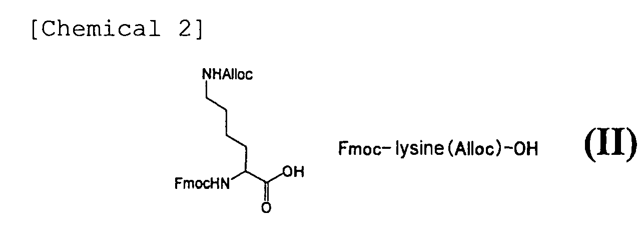

2. The method according to claim 1, wherein a different type of functional molecule is introduced after having introduced a functional molecule.

3. The method according to any of claim 1 and 2, wherein the functional molecule to be introduced is selected from a photoreactive functional molecule, membrane-permeating functional molecule, organ-selective functional molecule, bactericidal functional molecule, molecule-destroying functional molecule, adhesive functional molecule and molecule-recognizing functional molecule.

4. The method according to claim 2 or 3, wherein the functional molecule to be introduced contains a photofunctional molecule and a membrane-permeable functional molecule.

5. The method according to claim 4, wherein the photofunctional molecule is Cy3, Cy5, Bodipy, pyrene, naphthalimide, naphthaldiimide, FAM, FITC, ROX, TAMRA or Dabcyl, and the membrane-permeable functional molecule is a water-soluble amino acid derivative.

6. The method according to any of claims 1 to 5, wherein the protecting group that protects adenine, guanine, cytosine or thymine is a benzyloxycarbonyl group (Z group).

7. The method according to any of claims 1 to 6, wherein synthesis of PNA oligomer includes condensation and elongation in the PNA chain using solid-phase supports for the Boc method and Fmoc method.

8. The method according to any of claims 1 to 7, wherein the solid-phase support for the Boc method is methylbenzhydrylamine (MBHA) used for peptide synthesis in the solid-phase Boc method.

9. The method according to any of claims 1 to 7, wherein the solid-phase support for the Fmoc method is MBHA, a resin in which polystyrene is chloromethylated (Merrifield resin), a Merrifield resin modified with 4-hydroxybenzyl alcohol (Wang resin), an aminomethyl resin bonded with a Boc-amino acid linker (PAM resin), an aminomethyl resin bonded with an N-Fmoc-N-methoxy linker (Weinreb resin), a resin in which p-nitrobenzophenonoxime is bonded to polystyrene (Oxime resin) or a resin that has been tritylated using polystyrene (Trityl resin).

10. The method according to any of claims 1 to 9, wherein the introduction of a functional molecule having free carboxylic acid is carried out by dehydration condensation with a primary amino group obtained by selective de-protection by piperidine treatment of an Fmoc group in the Boc method or by zinc acetate solution treatment of an Alloc group in the Fmoc method.

11. The method according to claim 2 comprising the following a) to d),
one or two or more of a) and b):
a) production of a PNA oligomer by reacting a PNA monomer unit with Boc-lysine(Fmoc)-OH in a step of introducing Boc-lysine(Fmoc)- OH into a PNA oligomer;
b) introduction of a functional molecule into a PNA oligomer is carried out by dehydration condensation with a primary amino group obtained by selective de-protection by piperidine treatment of an Fmoc group in the aforementioned step of producing a functional PNA oligomer from a PNA oligomer; and
one or two or more of c) and d):
c) production of a PNA oligomer by reacting a PNA monomer unit with Fmoc-lysine(Alloc)-OH in a step of introducing Fmoc-lysine (Alloc)-OH into a PNA oligomer; and,
d) introduction of a functional molecule into a PNA oligomer is carried out by dehydration condensation with a primary amino group obtained by selective de-protection by zinc acetate solution treatment of an Alloc group in the aforementioned step of producing a functional PNA oligomer from a PNA oligomer.

12. A compound represented by the following general formula (III): 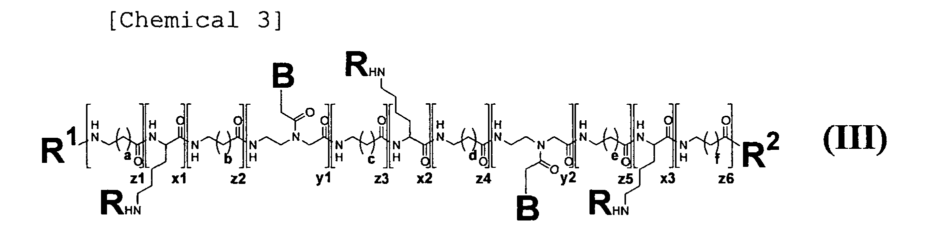
(wherein B's each independently represent adenine, guanine, cytosine or thymine, which may be the same or different, R's each independently represent an Fmoc group or a functional carboxylic acid derivative, which may be the same or different, R¹ represents a hydrogen atom or a functional carboxylic acid derivative, R² represents a derivative or a functional carboxylic acid derivative containing a hydrogen atom, an amino group, a hydroxyl group or a thiol group, a through f represent an integer from 0 to ∞, X₁ through X₃, Y₁, Y₂ and Z₁ through Z₆ all represent an integer of 0 or more, X₁ + X₂ + X₃ ≥ 0, Y₁ + Y₂ > 0 and Z₁ + Z₂ + Z₃ + Z₄ + Z₅ ≥ 0, provided that X₁ + X₂ + X₃ and Z₁ + Z₂ + Z₃ + Z₄ + Z₅ are not simultaneously 0, and in the case X₁ + X₂ + X₃ = 0, R¹ represents a functional carboxylic acid derivative).

13. The compound according to claim 12, wherein X₁ + X₂ + X₃ = 3 and Y₁ + Y₂ = 15.

14. The compound according to claim 13, wherein X₁ = 3 and Y₁ = 15.

15. The compound according to claim 14, wherein R or R¹ represents a cell membrane-permeable functional molecule derivative.

16. The compound according to claim 15, wherein R¹ represents a functional carboxylic acid derivative.

17. The compound according to claim 15 or 16, wherein X₁ = Z₁ = 1.

18. The compound according to any of claims 15 to 17, wherein Y₁ ≥ 2 and Z₂ = 1.

19. The compound according to any of claims 15 to 18, wherein a≤6, b≤4 and f ≤6.

20. The compound according to any of claims 15 to 19, wherein R¹ represents a photofunctional carboxylic acid derivative.
